# FASCICULE DE BREVET EUROPEEN

(11) **EP 2 904 969 B1**
(45) Date de publication et mention de la délivrance du brevet: **05.01.2022**
(21) Numéro de dépôt: 14191542.1
(22) Date de dépôt: 03.11.2014
(51) Int. Cl.: A61B 5/024, A61B 5/00, A61M 21/02

(54) **DISPOSITIF DE TRAITEMENT DU SYNDROME D'APNÉE DU SOMMEIL CHEZ UN PATIENT PAR STIMULATION KINESTHÉSIQUE**
VORRICHTUNG ZUR BEHANDLUNG DES SCHLAFAPNOE-SYNDROMS BEI EINEM PATIENTEN DURCH KINÄSTHETISCHE STIMULATION
DEVICE FOR TREATING THE SLEEP APNEA SYNDROME IN A PATIENT BY KINAESTHETIC STIMULATION

(30) Priorité: 11.02.2014 FR 1451060
(43) Date de publication de la demande: 12.08.2015
(73) Titulaire: Sorin CRM SAS, 92143 Clamart Cedex (FR); INSTITUT NATIONAL DE LA SANTE ET DE LA RECHERCHE MEDICALE (INSERM), 75013 Paris (FR); Université de Rennes 1, 35065 Rennes Cedex (FR); Université Grenoble Alpes, 38400 Saint-Martin-d'Hères (FR)
(72) Inventeur: Graindorge, Laurence, 44470 Thouaré sur Loire (FR); Amblard, Amel, 92330 Sceaux (FR); Henry, Christine, 75014 Paris (FR); Limousin, Marcel, 75014 Paris (FR); Duchemin Laporte, Laure, 91420 Morangis (FR); Hernandez, Alfredo, 35510 Cesson Sévigné (FR); Pepin, Jean-Louis, 38000 Grenoble (FR)
(74) Mandataire: Grünecker Patent- und Rechtsanwälte PartG mbB

(56) Documents cités:
- EP-A1- 1 741 387
- EP-A1- 2 465 434
- US-A1- 2012 016 431

## Description

L'invention concerne le diagnostic et le traitement des troubles du sommeil.

Elle concerne plus particulièrement l'utilisation à cet effet d'un dispositif dit de "stimulation kinesthésique", c'est-à-dire d'un dispositif de stimulation sensorielle externe du patient au moyen d'un vibreur en contact avec la peau dans une région sensible, précise du corps de ce patient. L'activation de ce vibreur a pour effet d'exciter localement les terminaisons cutanées ou mécano-récepteurs de la peau, et de déclencher une réponse du système nerveux autonome du patient, à prépondérance sympathique (ci-après "réponse autonomique").

La réponse autonomique à une activation sympathique est observable sur les principaux effets modulateurs de l'activité cardiaque, par exemple :
- effet chronotrope : augmentation de la fréquence cardiaque, ou diminution des intervalles RR ;
- effet inotrope : augmentation de la contractilité cardiaque.

Cette réponse autonomique est également observable sur la vasoconstriction périphérique, qui est augmentée en cas d'activation autonomique sympathique. Ce phénomène est notamment mis en œuvre avec le dispositif décrit par le US 2013/0102937 A1, qui propose de traiter l'hypertension par une stimulation appropriée de barorécepteurs ou de nerfs, notamment pendant les périodes où le patient est endormi. La stimulation est déclenchée quand le patient est reconnu comme étant en sommeil, puis maintenue ensuite à un niveau constant.

Outre ces conséquences sur l'activité cardiaque, une activation sympathique provoque des réponses sur le système respiratoire ou sur le système nerveux central (réveils autonomiques).

Il s'agit d'un procédé non invasif, qui permet d'agir sur un certain nombre de troubles du sommeil en alternative aux approches thérapeutiques classiques basées sur l'application d'une pression positive continue à travers un masque facial (traitement dit par CPAP), l'utilisation d'une orthèse de propulsion mandibulaire et/ou la stimulation électrique du nerf grand hypoglosse, qui implique la pose d'un implant de type stimulateur.

En particulier, la pathologie respiratoire connue sous le nom de "syndrome d'apnée du sommeil" (SAS) est caractérisée par la survenue fréquente (au moins 10 à 20 fois par heure) d'apnées pendant une phase de sommeil du patient, une "apnée" (ou pause respiratoire) étant définie comme un arrêt temporaire de la fonction respiratoire de durée supérieure à 10 secondes. Elle peut également être caractérisée par la survenue d'hypopnées dans les mêmes conditions, une "hypopnée" étant définie comme une décroissance importante (mais sans interruption) du débit respiratoire, typiquement une décroissance de plus de 50 % par rapport à une moyenne de référence antérieure. Dans la suite de la description, on ne distinguera pas ces deux phénomènes, une référence à des "apnées" étant censée inclure également les hypopnées.

Cette pathologie atteint plus de 4 % de la population et plus de 50 % des patients souffrant d'insuffisance cardiaque. Afin de protéger l'individu contre l'asphyxie liée à la diminution de la concentration en oxygène du sang pendant l'interruption ou la réduction du débit respiratoire, le corps s'adapte mais avec un effet délétère sur le sommeil, entraînant des microréveils inconscients. Il s'ensuit en phase d'éveil une somnolence diurne avec perte d'attention et un accroissement des risques d'accident de la route. Par ailleurs, plusieurs études ont montré chez les patients souffrant de SAS une plus grande incidence de troubles tels qu'hypertension artérielle, arythmies ventriculaires, infarctus myocardique et insuffisance cardiaque.

Plusieurs documents décrivent la possibilité de stopper les épisodes d'apnée grâce à un traitement par stimulation.

Ainsi, le US 2008/0009915 A1 propose une thérapie par stimulation acoustique, le US 2004/0215236 A1 par stimulation vestibulaire, le WO 2009/154458 A1 par stimulation électrique et le US 4 813 427 A par application d'un gaz en parallèle à une stimulation tactile.

La stimulation kinesthésique a déjà été proposée dans le passé pour minimiser ou interrompre les épisodes d'apnée du sommeil chez l'adulte ou chez le nouveau-né, comme cela est décrit par exemple dans le WO 2007/141345 A1 (FR 2 908 624 A1).

Ces techniques sont également décrites dans les articles de Pichardo R et al., "Validation of a Vibrotactile Stimulation System to Treat Apnea of Prematurity", Proceedings of the IEEE 27th annual Northeast Bioengineering Conférence, University of Connecticut, Storrs, CT (2001) 13-14, et de Beuchée A et al. "Stimulateur kinesthésique automatisé asservi à la détection d'apnées-bradycardies chez le nouveau-né prématuré", ITBMRBM, 28(2007) 124-130.

Le premier inconvénient de ces méthodes de stimulation est la variabilité des réponses en fonction du patient, ou même en fonction de l'état du patient et notamment du stade de sommeil. Cette variabilité peut nécessiter des méthodes complexes d'initialisation du système pour s'adapter aux paramètres du patient.

Le deuxième inconvénient est un phénomène d'accoutumance, qui oblige à faire évoluer constamment la thérapie pour qu'elle reste efficace. Certains systèmes décrivent une variation randomisée de la thérapie, qui risque alors d'être peu reproductible.

Enfin, le troisième inconvénient est le risque de réveiller le patient. En effet, les stimulations peuvent générer des réveils responsables à leur tour de la déstructuration du sommeil. Ces réveils font perdre en grande partie le bénéfice de la thérapie. Or peu de méthodes prennent en compte ce risque, ou bien décrivent une méthode peu réalisable car par exemple basée sur la détection des stades de sommeil par analyse d'un EEG, technique en pratique peu réalisable en routine ou à la maison.

Un traitement par stimulation kinesthésique de cette pathologie consiste, sur détection d'un épisode d'apnée ou d'hypopnée, à générer une stimulation appropriée susceptible de provoquer chez le patient une réponse autonomique propre à déclencher une modification respiratoire par exemple une reprise respiratoire ou une augmentation de la respiration qui mettra fin à l'apnée.

Ainsi, l'objectif de l'invention est de proposer une technique permettant d'interrompre les épisodes d'apnée par stimulation kinesthésique, en générant une réponse autonomique suffisante et adaptée pour arrêter ces apnées mais qui ne soit pas excessive, afin de limiter l'apparition de microréveils.

À cet égard, des études cliniques ont montré que, pour une énergie de stimulation donnée, la réponse autonomique est dépendante du stade de sommeil (stades de sommeil lent I à IV et sommeil paradoxal). Voir par exemple Hord D et al. "The Evoked Heart Rate Response during Sleep", Psychophysiology, 3:46-54 (1966), qui étudie divers paramètres de la variation de réponse autonomique de patients lorsque des stimuli sonores sont émis à proximité de leur oreille à différents moments du sommeil.

L'idée de base de l'invention est, au vu de cette relation entre le stade de sommeil et la réponse autonomique, i) de détecter le stade de sommeil courant et ii) d'asservir l'énergie de la stimulation kinesthésique au stade de sommeil courant ainsi détecté à l'instant où la stimulation est appliquée, de manière à générer une réponse autonomique suffisante pour arrêter les apnées mais dont le niveau reste suffisamment faible pour empêcher ou à tout le moins limiter l'apparition de microréveils.

Selon un premier aspect de l'invention, le problème est de trouver un moyen de déterminer automatiquement le stade de sommeil courant du patient, notamment (mais de façon non limitative) pour pouvoir appliquer une thérapie par stimulation kinesthésique à un niveau d'énergie approprié, comme on vient de l'exposer, ou bien toute autre thérapie (stimulation cardiaque, stimulation neuromusculaire...) de manière différenciée en fonction du stade de sommeil courant à l'instant de l'application de la thérapie.

Cette discrimination des stades de sommeil pourra être également utilisée à des fins simplement diagnostiques, notamment comme alternative moins coûteuse et plus commode à la polysomnographie (PSG), technique utilisée aujourd'hui pour analyser la structure du sommeil et diagnostiquer certaines pathologies du sommeil.

En effet, les stades de sommeil sont aujourd'hui évalués le plus souvent manuellement, parfois automatiquement, à partir d'enregistrements des signaux électroencéphalographiques (EEG) recueillis au cours d'un examen polysomnographique, par analyse de l'amplitude et de la fréquence d'au moins trois voies de signaux EEG. La difficulté est liée à la pose de nombreux capteurs EEG, qui rend un tel examen non réalisable en routine. D'autres méthodes de détection des stades du sommeil ont été proposées utilisant des enregistrements Holter cardiorespiratoires, à partir de la variabilité du rythme sinusal, etc., mais ces méthodes ne permettent que de différencier entre état d'éveil et état de sommeil, ou entre état REM (sommeil paradoxal) et état non-REM (sommeil à ondes lentes), elles ne permettent pas de distinguer tous les stades de sommeil, notamment de discriminer entre les différents stades de sommeil à ondes lentes, de I (somnolence) à IV (sommeil profond)

Une discrimination automatique des stades de sommeil pourra notamment permettre un meilleur diagnostic du sommeil, car pendant une PSG le patient dort mal dans la mesure où il se trouve dans une chambre d'hôpital et est couvert de toutes sortes de capteurs.

Il existe donc le besoin de disposer d'un système plus léger et automatique permettant donc à la fois au patient de mieux dormir et d'apporter au médecin une méthode plus rapide et moins couteuse, applicable facilement de façon routinière.

Un second aspect de l'invention a trait à l'utilisation de la connaissance du stade de sommeil courant - qu'il ait été déterminé conformément au premier aspect de l'invention ou par une autre technique - pour appliquer une thérapie par stimulation kinesthésique à un niveau d'énergie approprié, de manière différenciée en fonction du stade de sommeil courant à l'instant de l'application de la thérapie, afin de générer une réponse autonomique suffisante pour arrêter les apnées mais dont le niveau reste suffisamment faible pour empêcher ou à tout le moins limiter l'apparition de microréveils.

Plus précisément, selon le premier aspect précité, l'invention propose un dispositif de discrimination des stades successifs du sommeil d'un patient.

Ce dispositif est du type général comprenant, de manière en elle-même connue par exemple d'après les articles précités de Pichardo et al. et de Beuchée et al. : un générateur apte à produire des salves d'impulsions contrôlées de stimulation kinesthésique ; au moins un effecteur kinesthésique apte à être appliqué sur un site cutané externe du patient, et comprenant un transducteur électromécanique vibrant apte à recevoir les impulsions produites par le générateur ; des moyens de mesure d'au moins un paramètre témoin de l'activité autonomique courante du patient ; et des moyens détecteurs, aptes à déterminer un état de sommeil du patient.

Le dispositif comprend en outre, pour opérer la discrimination des stades successifs du sommeil, des moyens d'analyse du sommeil, activés conditionnellement seulement en présence d'un état de sommeil déterminé par les moyens détecteurs et comprenant : des moyens de commande, aptes à commander l'activation du générateur pour déclencher la production d'une salve d'impulsions kinesthésiques ; des moyens aptes à déterminer, en fonction de ladite mesure d'au moins un paramètre témoin de l'activité autonomique courante du patient, une variation de ce paramètre témoin consécutive à ladite production de la salve d'impulsions kinesthésiques ; des moyens évaluateurs, aptes à déterminer une réponse autonomique du patient à la stimulation kinesthésique en fonction de ladite variation. De façon caractéristique de l'invention, les moyens d'analyse du sommeil comprennent des moyens discriminateurs, aptes à déterminer, suite à la stimulation kinesthésique produite par l'activation du générateur par les moyens de commande, et en fonction de la réponse autonomique déterminée par les moyens évaluateurs, le stade de sommeil du patient parmi plusieurs stades prédéterminés du groupe comprenant : sommeil à ondes lentes I, sommeil à ondes lentes II, sommeil à ondes lentes III, sommeil à ondes lentes IV, et sommeil paradoxal REM.

Selon diverses caractéristiques subsidiaires avantageuses :
- le paramètre témoin de l'activité autonomique courante du patient est la fréquence cardiaque courante du patient, ou un paramètre du groupe comprenant : débit respiratoire ; saturation du sang en oxygène ; et paramètre dérivé d'un signal phonocardiographique ou d'accélération endocardiaque ;
- le dispositif comprend en outre des moyens d'initialisation aptes, sur détermination d'un état de sommeil par les moyens détecteurs, à établir le niveau d'au moins un paramètre de stimulation prédéterminé de la salve d'impulsions de stimulation kinesthésique, ce paramètre de stimulation étant un paramètre du groupe comprenant : énergie délivrée ; durée de la salve d'impulsions de stimulation ; fréquence de répétition des impulsions ; et durée unitaire des impulsions ;
- ces moyens d'initialisation comprennent des moyens de test comportant : des moyens aptes à initialiser le niveau du paramètre de stimulation à une valeur par défaut ; des moyens aptes à comparer la réponse autonomique courante déterminée par les moyens évaluateurs à un premier seuil ; et des moyens aptes, si la réponse autonomique courante est inférieure au premier seuil, à modifier par pas et de manière itérative le niveau du paramètre de stimulation jusqu'à franchissement du premier seuil ;
- le dispositif comprend en outre des moyens aptes à activer de manière réitérée les moyens d'analyse du sommeil et comprenant : des moyens aptes à comparer la réponse autonomique courante avec une valeur précédente, mémorisée, de la réponse autonomique ; et des moyens aptes à réactiver les moyens discriminateurs si l'écart courant entre la réponse autonomique courante et la réponse autonomique précédente excède un second seuil prédéterminé ;
- les moyens discriminateurs sont aptes à déterminer le stade de sommeil de manière relative, par rapport à un niveau précédent mémorisé du stade de sommeil du patient, en fonction de l'écart courant déterminé entre la réponse autonomique courante et la réponse autonomique précédente mémorisée ;
- ces moyens discriminateurs sont des moyens déterministes, aptes à élever le niveau du stade de sommeil en cas de diminution de l'écart entre la réponse autonomique courante et la réponse autonomique précédente, et *vice versa,* ou bien des moyens stochastiques mettant en œuvre un automate à nombre d'états fini dont les transitions sont définies par un processus markovien ou semi-markovien.

Selon le second aspect précité, l'invention propose un dispositif de traitement du syndrome d'apnée du sommeil chez un patient par stimulation kinesthésique, comprenant, de manière en elle-même connue par exemple d'après le US 2013/0102937 A1 : un générateur apte à produire des salves d'impulsions contrôlées de stimulation kinesthésique ; au moins un effecteur kinesthésique apte à être appliqué sur un site cutané externe du patient, et comprenant un transducteur électromécanique vibrant apte à recevoir les impulsions produites par le générateur et à délivrer une énergie de stimulation kinesthésique donnée ; et des moyens détecteurs, aptes à déterminer un état de sommeil du patient.

Le dispositif comprend en outre des moyens de commande adaptative du générateur, activés conditionnellement seulement en présence d'un état de sommeil déterminé par les moyens détecteurs.

De façon caractéristique de l'invention, les moyens de commande adaptative du générateur comprennent : des moyens discriminateurs, aptes à déterminer le stade de sommeil du patient parmi plusieurs stades prédéterminés du groupe comprenant : sommeil à ondes lentes I, sommeil à ondes lentes II, sommeil à ondes lentes III, sommeil à ondes lentes IV, et sommeil paradoxal REM ; et des moyens modulateurs, aptes à modifier (70-72) le niveau de l'énergie de stimulation kinesthésique des salves produites par le générateur en fonction du stade de sommeil du patient déterminé par les moyens discriminateurs.

Selon diverses caractéristiques subsidiaires avantageuses :
- les moyens modulateurs sont aptes à augmenter l'énergie de stimulation pour un stade de sommeil plus élevé, et *vice versa* ;
- le dispositif comprend en outre des moyens de détection de la survenue d'un épisode d'apnée ou d'hypopnée et des moyens aptes à activer le générateur conditionnellement sur détection d'un épisode d'apnée ou d'hypopnée, ainsi qu'éventuellement des moyens de détection de la fin de l'épisode d'apnée ou hypopnée et des moyens aptes à désactiver le générateur sur détection de la fin de l'épisode d'apnée ou hypopnée ;
- les moyens modulateurs comprennent des moyens aptes à appliquer un incrément/décrément prédéterminé d'énergie de stimulation fonction de chacun des stades de sommeil prédéterminés ;
- les moyens modulateurs comprennent une table de correspondance attribuant un niveau d'énergie de stimulation prédéterminé à chacun des stades de sommeil prédéterminés, ainsi qu'éventuellement des moyens d'initialisation aptes, sur détermination d'un état de sommeil par les moyens détecteurs, à établir les valeurs de niveau d'énergie de la table de correspondance ;
- le dispositif comprend en outre des moyens évaluateurs, aptes à déterminer une réponse autonomique du patient à la stimulation kinesthésique en fonction de la variation, consécutive à la production de la salve d'impulsions, d'un paramètre témoin de l'activité autonomique courante du patient ;
- le paramètre témoin de l'activité autonomique courante du patient est la fréquence cardiaque courante du patient ou un paramètre du groupe comprenant : débit respiratoire ; saturation du sang en oxygène ; et paramètre dérivé d'un signal phonocardiographique ou d'accélération endocardiaque ;
- les moyens d'initialisation comprennent, pour établir la valeur de niveau d'énergie correspondant au premier stade de sommeil : des moyens aptes à initialiser l'énergie de stimulation à une valeur minimale par défaut prédéterminée ; des moyens aptes à comparer la réponse autonomique courante déterminée par les moyens évaluateurs à un premier seuil ; des moyens aptes, si la réponse autonomique courante est inférieure au premier seuil, à modifier par pas et de manière itérative l'énergie de stimulation jusqu'à franchissement du premier seuil ; et des moyens aptes à mémoriser, pour le premier stade de sommeil, la valeur de l'énergie de stimulation obtenue après franchissement du seuil ;
- le dispositif comprend en outre des moyens aptes à calculer et à mémoriser des valeurs d'énergie de stimulation correspondant aux stades de sommeil suivants le premier stade, en fonction de la valeur d'énergie de stimulation mémorisée pour le premier stade ;
- les moyens discriminateurs sont aptes à déterminer le stade de sommeil courant en fonction de la réponse autonomique déterminée par les moyens évaluateurs.

On va maintenant décrire un exemple de mise en œuvre du dispositif de l'invention, en référence aux dessins annexés où les mêmes références numériques désignent d'une figure à l'autre des éléments identiques ou fonctionnellement semblables.
La Figure 1 illustre de façon générale les différents stades successifs au cours d'une nuit de sommeil d'un patient.
La Figure 2 illustre schématiquement les principaux composants d'un système de stimulation kinesthésique selon l'invention.
La Figure 3 illustre les variations de la réponse autonomique à une impulsion de stimulation kinesthésique, cette réponse étant évaluée par les variations de la fréquence cardiaque du patient.
La Figure 4 est une représentation illustrant les variations de la réponse autonomique d'une population de patients à différents stades de sommeil, pour une même énergie de stimulation kinesthésique.
La Figure 5 est une représentation illustrant la proportion de microréveils effectifs dans une population de patients en fonction des différents stades de sommeil, pour une même énergie, maximale, de stimulation kinesthésique.
Les Figures 6 et 7 sont des organigrammes illustrant les différentes étapes mises en œuvre par la technique selon l'invention de détection automatique des stades de sommeil.
Les Figures 8 et 9 sont des organigrammes illustrant les différentes étapes successives mises en œuvre par la technique selon l'invention d'asservissement de la stimulation kinesthésique en fonction du stade de sommeil.

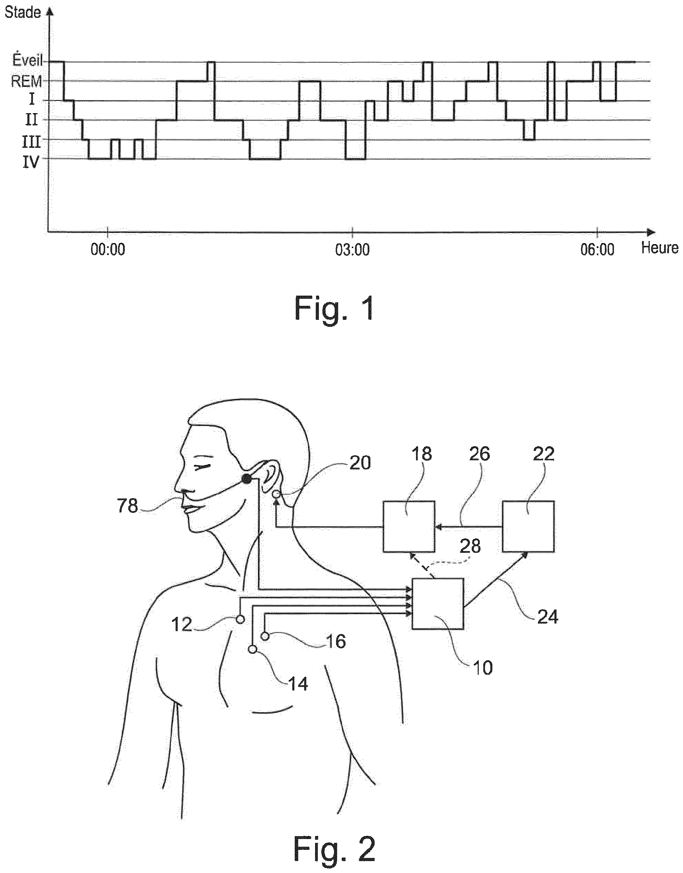

La Figure 1 est un diagramme montrant les différents stades de sommeil successifs d'un patient au cours d'une nuit de sommeil.

Ce sommeil se présente sous la forme d'une succession de cycles au cours desquels, à partir de l'état d'éveil, le patient entre dans des cycles de sommeil à ondes lentes de plus en plus profonds, des stades I (somnolence) et II (sommeil léger) jusqu'aux stades III et IV (sommeil profond). Ensuite, généralement après un court retour en stade I le patient entre dans un stade dit de sommeil paradoxal, également désigné stade REM (pour *Rapid Eye Movements),* qui est caractérisé par une forte activité électrique du cerveau. Les cycles se succèdent ainsi jusqu'à l'éveil final matinal.

Les différents stades correspondent à des formes d'activité cérébrale distinctes caractérisées par des tracés EEG spécifiques, reconnaissables sur un enregistrement effectué par exemple lors d'un examen polysomnographique.

Le déroulement de la période de sommeil du patient peut être entrecoupé de microréveils. Selon l'importance du microréveil, le patient pourra soit retourner au même stade, soit redescendre d'un stade ou repasser directement vers un stade de sommeil plus léger.

L'un des buts de l'invention est de proposer une technique non invasive de détermination, pratiquement en temps réel, du stade de sommeil courant d'un individu endormi, sans recours à un examen EEG (donc sans mise en place d'une multitude d'électrodes sur la tête du patient) et avec un risque minimal ou nul de provoquer des microréveils qui seraient délétères.

Un autre but de l'invention est d'utiliser cette information de stade de sommeil courant pour moduler une stimulation kinesthésique, de manière notamment à mettre fin à des épisodes d'apnée du sommeil (ou d'hypopnée), sans pour autant provoquer un microréveil du patient, qui aurait des conséquences négatives faisant perdre tout bénéfice à la suppression des apnées.

La Figure 2 illustre schématiquement les principaux composants d'un système utilisable à cet effet.

Ce système comporte un enregistreur Holter 10 relié à divers capteurs ou électrodes 12, 14, 16, permettant de mesurer des signaux physiologiques tels que la fréquence cardiaque, la respiration, la saturation en oxygène, l'onde de pouls, le phonocardiogramme, etc. Dans la suite, on s'intéressera principalement à la fréquence cardiaque, qui est un paramètre simple à obtenir, mais cette mesure n'est pas limitative et l'invention peut être mise en œuvre à partir d'autres signaux physiologiques recueillis sur le patient. Le système comprend par ailleurs un dispositif de stimulation kinesthésique, avec un boîtier générateur 18 produisant des impulsions appliquées à un effecteur de stimulation kinesthésique 20, constitué par exemple d'un vibreur placé dans une région sensible de l'épiderme, typiquement (chez l'adulte) dans la région de l'os mastoïde au voisinage de l'oreille. La stimulation vibrotactile appliquée sur la peau par l'effecteur 20 est détectée par les récepteurs sensoriels ou mécanorécepteurs de l'organisme, et cette information est ensuite transmise par l'intermédiaire des nerfs sensitifs au système nerveux central autonome.

L'effecteur 20 est par exemple un transducteur du type *C10-100* de Precision Microdrives ou *C2 Tactor* de Engineering Acoustics. Il s'agit d'un transducteur de quelques grammes susceptible d'émettre des vibrations grâce à un vibreur intégré excité par des trains d'impulsions d'amplitude et de durée variables, typiquement à une fréquence de 250 Hz qui est la fréquence nominale de résonance de cet effecteur particulier et qui est également la fréquence à laquelle les mécanorécepteurs de la peau sont les plus sensibles. D'autres types d'effecteur peuvent bien entendu être efficacement utilisés.

Le boitier de commande 18 est piloté par un microcontrôleur et possède des moyens permettant de régler l'intensité (c'est-à-dire l'énergie) de la stimulation kinesthésique, par variation contrôlée de l'amplitude et/ou du nombre, de la durée et/ou de la fréquence de stimulation des trains d'impulsions formant le signal appliqué à l'effecteur 20.

Le système comprend également un boitier 22 couplé au dispositif Holter 10 et au boitier de commande 18 par une liaison filaire ou sans fil respective 24, 26, de manière à recevoir des données du dispositif Holter 10, traiter ces données et produire en réponse des informations de contrôle de la stimulation kinesthésique qui seront transmises au boitier de commande 18. En variante, le traitement des données et le pilotage du boitier de commande 18 peuvent être opérés au sein même du dispositif Holter 10 et transmis directement par une liaison 28 au boitier 18.

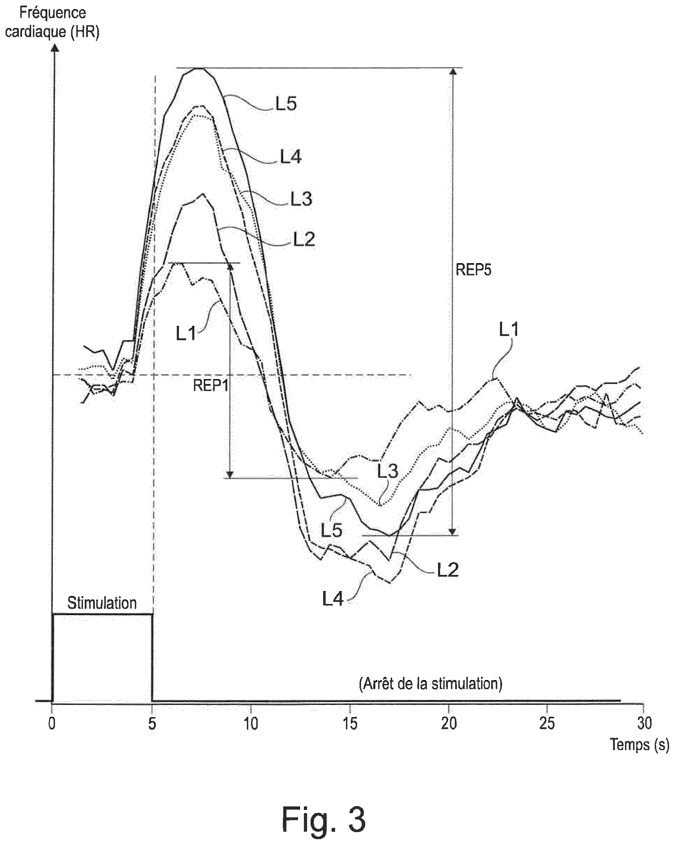

La Figure 3 illustre, chez un patient donné, la réponse autonomique à une stimulation kinesthésique, exprimée ici en termes de variations de la fréquence cardiaque.

Une telle mesure est aisée à obtenir mais n'est pas limitative de l'invention, qui peut être mise en œuvre avec d'autres techniques de quantification de la fonction autonomique, par exemple à partir de la pression sanguine ou des bruits du cœur (par un capteur phonocardiographique ou un capteur d'accélération endocardiaque). L'invention s'appuie sur l'hypothèse qu'une stimulation kinesthésique appliquée dans une région sensible de l'épiderme telle que de préférence la région de l'oreille permet de stimuler les voies autonomiques sans pour autant générer de réveil du patient : on peut ainsi provoquer une réponse, mesurable, à une stimulation du système autonomique.

Ainsi, dans une étude sur des patients souffrant de SAS on a pu comparer les données de deux nuits d'enregistrement PSG : de façon randomisée, le patient passait une nuit sans stimulation kinesthésique et l'autre nuit avec stimulation kinesthésique d'énergie variable et appliquée à intervalles réguliers. L'analyse de ces résultats a montré que lors de nuits avec stimulation on observait plus de microréveils autonomes, montrant l'efficacité de la stimulation à activer le système autonomique. D'autre part, la durée de sommeil et la durée des différents stades n'étaient pas significativement différentes, montrant que la stimulation n'engendrait pas de déstructuration du sommeil.

On a représenté sur la Figure 3 les variations de fréquence cardiaque moyenne relevées pour différents niveaux de stimulation kinesthésique appliqués à un patient donné pendant son sommeil (tous stades de sommeil confondus).

À t = 0, une salve d'impulsions de stimulation est appliquée, à un niveau d'énergie donné, puis arrêtée à t = 5 s.

Les variations de la fréquence cardiaque induites par cette stimulation, représentatives de la réponse autonomique du patient, sont illustrées par les caractéristiques L1 à L5, qui correspondent à des énergies de stimulation croissantes.

De façon caractéristique, la réponse est une courbe biphasique, avec une augmentation de la fréquence cardiaque suivie d'une diminution au-dessous du niveau de base initial, puis retour à une fréquence à peu près stable au bout d'une vingtaine de cycles après la fin du stimulus.

Cette réponse biphasique s'explique par une activation initiale sympathique due à la stimulation (accélération de la fréquence cardiaque), suivie d'une réponse de compensation parasympathique (courbe de décélération). On peut observer aussi que la réponse autonomique, mesurée sur les caractéristiques L1 ... L5 par les amplitudes respectives REP1 ... REP5 correspondant à l'excursion maximale de la fréquence cardiaque après stimulation, croît avec l'énergie de cette stimulation. L'observation de la variation de fréquence post-stimulation permet donc d'évaluer l'importance de la réponse autonomique du patient.

L'invention a pour objet d'utiliser la réponse autonomique mesurable à deux fins :
- la détermination du stade de sommeil à un moment donné ; et
- en fonction du stade de sommeil ainsi déterminé, en cas d'apnée du sommeil détectée, l'application d'une stimulation kinesthésique pour arrêter l'apnée, mais dont l'énergie est modulée en fonction du stade de sommeil de manière à ne pas provoquer chez le patient de microréveils.

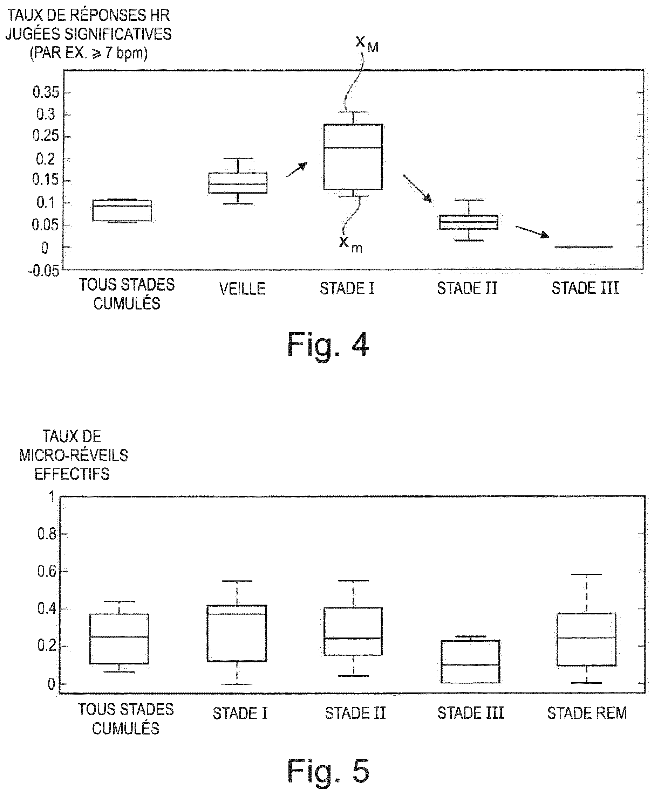

Les Figures 4 et 5 montrent, sous forme de *box-plots* ou "boites à moustaches", les résultats d'une étude faite sur une population de patients sur lesquels a été évaluée l'importance de cette réponse autonomique (mesurée à partir de la variation de la fréquence cardiaque) en réponse à l'application d'une impulsion de stimulation kinesthésique, dans diverses circonstances.

Sur la Figure 4, on a porté en ordonnée le taux de réponses jugées significatives dans la population de patients (par exemple les réponses autonomiques produisant une excursion de fréquence cardiaque d'au moins 7 bpm) respectivement dans les situations suivantes : tous stades de sommeil cumulés, à l'état de veille, à l'état de sommeil au stade I, à l'état de sommeil au stade II, et à l'état de sommeil au stade III. On notera incidemment que, s'agissant d'une population de patients souffrant de troubles respiratoires de type apnée du sommeil, aucun n'atteint le stade IV le plus profond.

Dans chacune de ces situations, le trait central représente la médiane des échantillons, et sa position dans la boite permet d'apprécier la symétrie des données. Les lignes inférieure et supérieure de la boite représentent respectivement les quantiles empiriques d'ordre p= 1/4 (premier quartile) et p= 3/4 (dernier quartile). La hauteur de la boite est donc le rang interquartile. Les deux lignes des limites supérieure et inférieure montrent les valeurs maximale *x_{M}* et minimale *xₘ* relevées pour le cas considéré. Cette Figure 4 montre que, pour des différents stades de sommeil, l'importance de la réponse autonomique suite à une stimulation kinesthésique diminue lorsque le stade de sommeil devient plus profond : la réponse au stade II est plus faible que celle au stade I, et celle au stade III (stade qui n'a d'ailleurs été atteint que par un seul patient dans la population étudiée) étant plus faible que celle au stade II.

En d'autres termes, pour déclencher une même réponse autonomique, par exemple pour mettre fin à un même épisode d'apnée, l'énergie de stimulation kinesthésique nécessaire devra être d'autant plus élevée que le stade de sommeil est plus profond.

Sur la Figure 5 on a illustré, avec les mêmes conventions que précédemment, les valeurs des taux de microréveils (déterminés par une analyse polysomnographique par exemple) pour les mêmes stades que ceux de la Figure 5 : tous stades de sommeil cumulés, stades I, II, III et REM.

On voit que le taux de microréveils dépend également du stade de sommeil : à une énergie donnée, plus le stade de sommeil correspond à un sommeil profond, plus le taux de microréveils diminue. Il sera donc possible d'augmenter l'énergie de la stimulation kinesthésique lorsque le stade de sommeil passera à un niveau plus profond, sans pour autant risquer d'augmenter les microréveils. En d'autres termes, l'énergie de stimulation pourra être augmentée lorsque le stade augmente, afin de garantir l'efficacité de la réponse ; inversement, dès qu'un stade léger sera de nouveau détecté, l'énergie de stimulation devra être de nouveau diminuée pour éviter de provoquer un réveil du patient.

### Détermination du stade de sommeil courant

On va maintenant exposer la manière dont le stade de sommeil du patient est déterminé à un moment donné.

Comme on l'a indiqué en introduction, cette détermination peut être avantageusement utilisée pour la thérapie de l'apnée du sommeil, en modulant de façon appropriée une stimulation kinesthésique pour ne pas provoquer de microréveils.

Cette utilisation n'est cependant pas limitative, et la détection des stades de sommeil pourra être utilisée à d'autres fins thérapeutiques ou même diagnostiques, par exemple pour une analyse par des moyens simples du déroulement d'une nuit de sommeil chez un patient afin de disposer d'un enregistrement des stades successifs au cours du temps, notamment pour vérifier si le patient atteint les stades de sommeil les plus profonds, les plus réparateurs, ou si un trouble sous-jacent l'empêche d'atteindre ces stades.

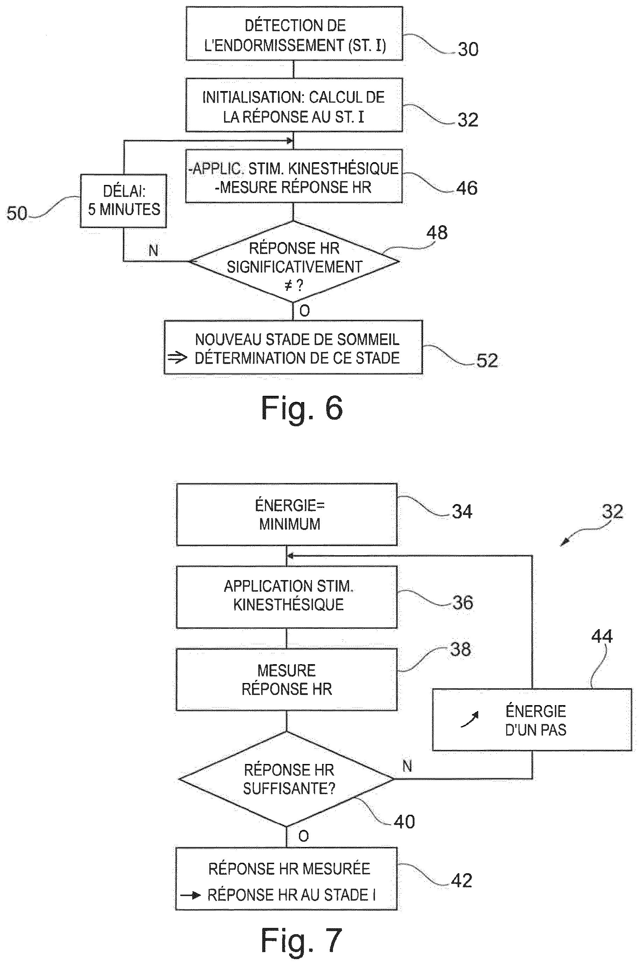

On va décrire en référence aux Figures 6 et 7 un exemple de mise en œuvre du procédé, selon l'invention, de détermination automatique des stades de sommeil en fonction de la réponse autonomique à une stimulation kinesthésique.

L'algorithme commence par une recherche de l'état du patient, pour détecter l'endormissement de celui-ci, c'est-à-dire le passage de l'état éveillé à un sommeil en stade I (étape 30). Cette détection de l'état éveil/sommeil du patient peut être opérée par exemple par une technique connue de recoupement d'informations délivrées par un capteur physiologique (capteur de ventilation-minute MV) et un capteur d'activité (capteur accélérométrique G), avec suivi de la fréquence cardiaque. Une telle technique est par exemple décrite dans le EP 1 317 943 A1 (ELA Medical).

Une fois l'endormissement détecté, le patient se trouve nécessairement en stade I. Il convient alors de procéder à une étape d'initialisation de la réponse autonomique correspondant à ce premier stade (étape 32). Cette initialisation de l'étape 32 est décrite plus en détail à la Figure 7 : tout d'abord, l'énergie de stimulation kinesthésique est initialisée au niveau minimum permis par l'effecteur de stimulation kinesthésique (étape 34). Une première stimulation est appliquée (étape 36) et la réponse autonomique est mesurée, typiquement par mesure de l'excursion de la fréquence cardiaque (HR) suite à l'application de l'impulsion (étape 38). Si la variation de la fréquence cardiaque est suffisante (test 40), par exemple d'au moins 7 bpm, pour qu'elle résulte d'une variation de la réponse autonomique à ce stade et au stade suivant - car la réponse autonomique diminuera quand le stade augmentera - alors l'énergie appliquée est mémorisée et conservée pour la nuit et la réponse HR mesurée est enregistrée comme correspondant à la réponse du stade I (étape 42). Dans le cas où la réponse observée n'est pas suffisante, alors l'énergie est augmentée d'un pas (étape 44) et le processus est réitéré (étapes 36 et suivantes) avec cette nouvelle énergie de stimulation. Dans une autre forme de mise en oeuvre, l'étape 36 peut comprendre un groupe de stimulations, afin d'obtenir une réponse autonomique moyenne, ou des stimulations à deux énergies différentes, afin de vérifier l'obtention d'une variation de fréquence entre les différentes énergies.

Si l'on revient maintenant à la Figure 6, après l'initialisation de l'étape 32, périodiquement ou lorsqu'il est nécessaire d'estimer le stade de sommeil courant, la stimulation kinesthésique est appliquée avec l'énergie qui vient d'être déterminée, et la réponse autonomique est mesurée (étape 46). Si la réponse HR n'a pas changé de manière significative (test 48), aucune action particulière n'est entreprise et le processus sera réitéré périodiquement (typiquement toutes les cinq minutes, délai de l'étape 50). De la même façon que pour l'étape 36, l'étape 46 peut comprendre également un groupe de stimulations, l'évaluation de l'étape 48 se faisant alors sur la moyenne des réponses observées.

Si, en revanche, le niveau de la réponse autonomique a varié de façon significative (test 48) par rapport au niveau précédemment testé et mémorisé, ceci indique que le stade de sommeil a vraisemblablement changé et que le nouveau stade doit être déterminé (étape 52).

Le nouveau stade de sommeil est déterminé à la fois i) en fonction de la réponse HR, c'est-à-dire de la variation de la fréquence cardiaque observée en réponse à la stimulation et ii) en fonction de l'historique récent des stades de sommeil par lesquels est passé le patient.

À cet effet, dans une première approche de type déterministe on considèrera qu'une diminution de la réponse HR révèle le passage dans un sommeil plus profond, alors qu'une augmentation de cette réponse révèle un passage en sommeil plus léger. L'importance de cette diminution/augmentation peut être également utilisée pour évaluer le stade de sommeil sur la base de données résultant d'études statistiques effectuées sur une population de patients, études qui ont permis d'évaluer des amplitudes de diminution/augmentation typiques en fonction des différentes transitions stade I/stade II, stade II/stade III, stade III/stade I, etc. Les données issues d'une étude préliminaire on ainsi montré que la réponse HR baisse en moyenne de 18 % entre le stade I et II, de 20 % entre le stade I et III, et de 10 % entre le stade I et le stade REM. En revanche, un passage à l'état de veille provoque une augmentation de 22 % de la réponse HR.

Une autre approche possible est une approche de type stochastique, markovienne ou semi-markovienne.

Les modèles de Markov cachés sont des automates à nombre d'états fini qui décrivent un système de manière stochastique et non déterministe. La structure de base d'un modèle de Markov consiste en un ensemble d'états S = (S1, S2... SN) connectés entre eux par des probabilités définies dans une table de transition. L'adjectif "caché" traduit ici le fait que l'émission des observations, à partir d'un état, suit une loi aléatoire et que le processus sous-jacent (le stade de sommeil) n'est pas directement observable (il est "caché"). C'est ce caractère aléatoire des mesures qui, ajouté aux propriétés des processus markoviens, fait la souplesse et la puissance de cette approche. Dans le cas des modèles du premier ordre, l'état du système à un instant t dépend uniquement de l'état du système à l'instant *t*-1, ce qui définit un processus markovien pur. Les modèles semi-markoviens cachés sont semblables aux modèles markoviens, mais l'état du système au temps t dépend non seulement de l'état à *t*-1, mais aussi d'autres paramètres, comme la durée de séjour dans l'état actuel. Dans l'approche proposée ici, chaque état du modèle markovien ou semi-markovien représente un stade de sommeil et les probabilités de transition d'un stade à l'autre dépendent à la fois des observables (réponse autonomique) et d'une matrice de probabilités de transition apprise à partir d'une base de données, comme celle déjà constituée dans les études citées plus haut. Ce type de modèle permet donc d'estimer, au vu des observables et de la matrice de transition apprise, l'état d'un système à tout instant t.

### Asservissement de la stimulation kinesthésique

### en fonction des stades de sommeil

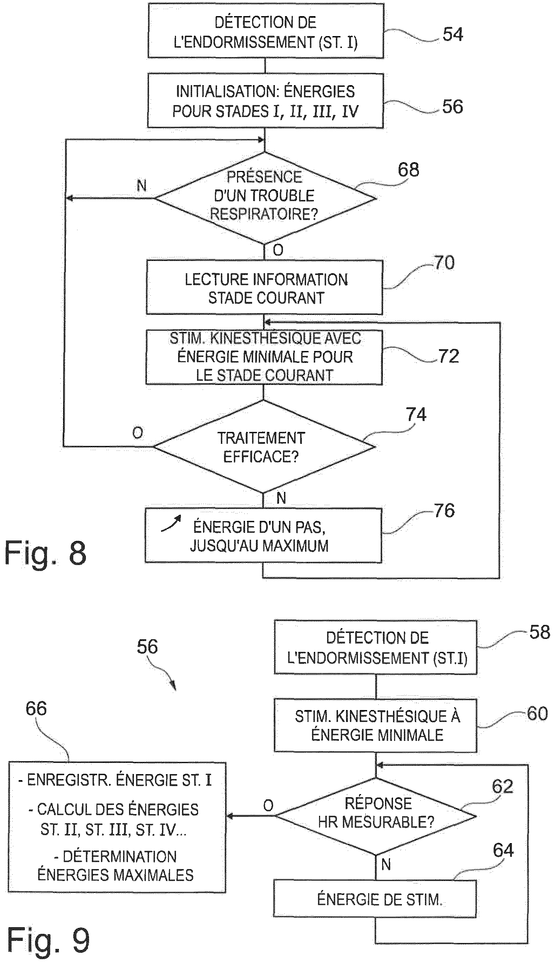

L'algorithme des Figures 8 et 9 illustre un exemple de traitement des troubles respiratoires de type apnée du sommeil ou hypopnée par une stimulation kinesthésique modulée en fonction du stade de sommeil courant du patient, selon le procédé de l'invention.

L'algorithme commence par la détection de l'endormissement du patient (étape 54), selon une technique comparable à ce qui a été décrit plus haut à propos de l'étape 30 de la Figure 6 pour la discrimination entre les différents stades de sommeil.

Une fois l'endormissement détecté, le patient se trouve alors en stade I, et les énergies de stimulation des différents stades sont alors initialisées (étape 56).

Une première technique peut consister à donner des valeurs fixes, croissantes au fur et à mesure que les stades sont plus profonds, aux énergies de stimulation des différents stades. Ces valeurs peuvent être identiques pour tous les patients (valeurs calculées à partir des moyennes des observations cliniques), ou peuvent être individualisées après évaluation préliminaire au cours d'un examen polysomnographique.

On peut également initialiser ces valeurs à partir d'une première énergie efficace (calcul de l'étape 42 de la figure 7) et ensuite calculer des variations en variation relative (pourcentage) ou absolue ("delta") de cette première valeur pour les autres stades de sommeil, ces variations étant directement tirées des observations cliniques.

Une autre technique consiste à appliquer la stimulation minimale telle que déterminée à l'étape 42 de la figure 7. Lorsque l'on détecte que le stade a changé, l'énergie de cette stimulation doit être modifiée : si le stade est plus profond, il faut augmenter l'énergie jusqu'à retrouver une variation significative du paramètre fréquence cardiaque ; si le stade est plus léger, il faut repartir de l'énergie minimum et remonter.

Plus précisément, l'étape d'initialisation est illustrée Figure 9, une fois l'endormissement détecté (étape 58, identique à l'étape 54 de la Figure 8) une première stimulation est appliquée, à l'énergie minimale (étape 60). Si une réponse mesurable n'est pas détectée (test 62), alors l'énergie de stimulation est augmentée d'un pas (étape 64) et la stimulation est réitérée jusqu'à trouver une réponse mesurable. L'énergie ainsi ajustée est enregistrée comme étant l'énergie efficace correspondant au stade I (étape 66). Les énergies correspondant aux autres stades sont déterminées à partir de cette valeur, soit en lui ajoutant une valeur fixe soit en l'augmentant d'un taux prédéterminé. Lors de la phase d'initialisation 56, on peut également établir la valeur maximale d'énergie de stimulation au-delà de laquelle un réveil est à craindre, et ceci pour chaque stade de sommeil. Cette valeur maximale pourra être dérivée des énergies évaluées pour les différents stades en leur ajoutant une marge prédéterminée, ou bien avoir été déterminée au cours d'un examen polysomnographique préalable.

Si l'on revient à la Figure 8, une fois l'initialisation terminée (étape 56), l'algorithme passe dans une phase de recherche de l'apparition d'un trouble respiratoire (étape 68).

Diverses méthodes de détection de l'apparition d'une apnée ou hypopnée ont été décrites, par exemple dans les EP 1 319 421 A1, EP 1 433 496 A1 ou EP 1 584 288 A1, tous trois au nom de ELA Medical. On pourra se référer à ces documents pour plus de détails sur la manière d'opérer la détection et le diagnostic de ces troubles du sommeil.
il est également possible, si le patient n'est pas équipé d'un implant, d'utiliser une canule nasale (78 sur la figure 2) comportant un capteur approprié permettant de détecter directement l'interruption du flux respiratoire normal.

Dès qu'un trouble est détecté, l'algorithme détermine le stade de sommeil courant (étape 70), information qui a été obtenue notamment par mise en oeuvre de l'algorithme décrit plus haut en référence aux Figures 6 et 7. Une stimulation kinesthésique est alors délivrée (étape 72) avec l'énergie qui avait été déterminée, pour le stade de sommeil en cours, lors de l'étape d'initialisation 56.

L'efficacité de la stimulation kinesthésique est alors évaluée (étape 74), c'est-à-dire que le dispositif détermine si la thérapie qui vient d'être appliquée a été ou non efficace.

Cette efficacité peut être évaluée en cours d'événement, immédiatement, c'est-à-dire que l'on regarde si l'on est en présence d'un épisode caractéristique de début d'apnée suivi d'une reprise rapide de la respiration révélant l'arrêt de l'apnée consécutif à la stimulation kinesthésique.

Une autre manière d'évaluer l'efficacité de la thérapie consiste, en variante ou en complément, à opérer un comptage du nombre des évènements révélateurs d'un trouble respiratoire sur une période donnée, par exemple de 5 ou 10 minutes, et à vérifier si, par rapport à un historique, ce comptage indique une diminution de la sévérité des symptômes ou non.

En tout état de cause, si la thérapie n'a pas été efficace, l'énergie de la stimulation est augmentée d'un pas (étape 76), et ceci jusqu'à un maximum prédéterminé correspondant à la limite risquant de provoquer un microréveil, donc avec risque que le traitement produise lui-même des effets délétères.

## Revendications

1. Un dispositif de traitement du syndrome d'apnée du sommeil chez un patient, comprenant :
- un générateur (18) apte à produire des salves d'impulsions contrôlées de stimulation kinesthésique ;
- au moins un effecteur (20) apte à être appliqué sur un site cutané externe du patient, et comprenant un transducteur électromécanique ;
- des moyens détecteurs, aptes à déterminer un état de sommeil du patient ; et
- des moyens de commande adaptative du générateur, activés conditionnellement seulement en présence d'un état de sommeil déterminé par les moyens détecteurs; et
- des moyens discriminateurs, aptes à déterminer (30-52) le stade de sommeil du patient parmi plusieurs stades prédéterminés du groupe comprenant : sommeil à ondes lentes I, sommeil à ondes lentes II, sommeil à ondes lentes III, sommeil à ondes lentes IV, et sommeil paradoxal REM,
**caractérisé en ce que** ledit effecteur est un effecteur kinesthésique (20), ledit transducteur électromécanique étant un transducteur vibrant apte à recevoir les impulsions produites par le générateur et à délivrer une énergie de stimulation kinesthésique donnée,
et **en ce que** les moyens de commande adaptative du générateur comprennent :
• des moyens modulateurs, aptes à modifier (70-72) le niveau de l'énergie de stimulation kinesthésique des salves produites par le générateur en fonction du stade de sommeil du patient déterminé par les moyens discriminateurs.

2. Le dispositif de la revendication 1, dans lequel les moyens modulateurs sont aptes à augmenter l'énergie de stimulation pour un stade de sommeil plus profond dans l'ordre croissant des stades successifs I, II, III, IV et REM, et *vice versa.*

3. Le dispositif de la revendication 1, comprenant en outre :
- des moyens de détection (68) de la survenue d'un épisode d'apnée ou d'hypopnée ; et
- des moyens aptes à activer le générateur conditionnellement sur détection d'un épisode d'apnée ou d'hypopnée.

4. Le dispositif de la revendication 3, comprenant en outre :
- des moyens de détection (68) de la fin de l'épisode d'apnée ou hypopnée ; et
- des moyens aptes à désactiver le générateur sur détection de la fin de l'épisode d'apnée ou hypopnée.

5. Le dispositif de la revendication 1, dans lequel les moyens modulateurs comprennent des moyens aptes à appliquer un incrément/décrément prédéterminé d'énergie de stimulation fonction de chacun des stades de sommeil prédéterminés.

6. Le dispositif de la revendication 1, dans lequel les moyens modulateurs comprennent une table de correspondance attribuant un niveau d'énergie de stimulation prédéterminé à chacun des stades de sommeil prédéterminés.

7. Le dispositif de la revendication 6, comprenant en outre des moyens d'initialisation (56) aptes, sur détermination d'un état de sommeil par les moyens détecteurs, à établir les valeurs de niveau d'énergie de la table de correspondance.

8. Le dispositif de la revendication 1, comprenant en outre :
- des moyens évaluateurs, aptes à déterminer une réponse autonomique du patient à la stimulation kinesthésique en fonction de la variation, consécutive à la production de la salve d'impulsions, d'un paramètre témoin de l'activité autonomique courante du patient.

9. Le dispositif de la revendication 8, dans lequel le paramètre témoin de l'activité autonomique courante du patient est la fréquence cardiaque courante (HR) du patient.

10. Le dispositif de la revendication 8, dans lequel le paramètre témoin de l'activité autonomique courante du patient est un paramètre du groupe comprenant : débit respiratoire ; saturation du sang en oxygène ; et paramètre dérivé d'un signal phonocardiographique ou d'accélération endocardiaque.

11. Le dispositif de la revendication 8, dans lequel les moyens d'initialisation (56) comprennent, pour établir la valeur de niveau d'énergie correspondant au premier stade de sommeil :
- des moyens aptes à initialiser (60) l'énergie de stimulation à une valeur minimale par défaut prédéterminée ;
- des moyens aptes à comparer (62) la réponse autonomique courante déterminée par les moyens évaluateurs à un premier seuil ;
- des moyens aptes, si la réponse autonomique courante est inférieure au premier seuil, à modifier (64) par pas et de manière itérative l'énergie de stimulation jusqu'à franchissement du premier seuil ; et
- des moyens aptes à mémoriser (66), pour le premier stade de sommeil, la valeur de l'énergie de stimulation obtenue après franchissement du seuil.

12. Le dispositif de la revendication 11, comprenant en outre des moyens aptes à calculer (66) et à mémoriser des valeurs d'énergie de stimulation correspondant aux stades de sommeil suivants le premier stade, en fonction de la valeur d'énergie de stimulation mémorisée pour le premier stade.

13. Le dispositif de la revendication 8, dans lequel les moyens discriminateurs sont aptes à déterminer (30-52) le stade de sommeil courant en fonction de la réponse autonomique déterminée par les moyens évaluateurs.

## Patentansprüche

1. Vorrichtung zur Behandlung des Schlafapnoe-Syndroms bei einem Patienten, umfassend :
- einen Generator (18), der geeignet ist, Bursts von kontrollierten Impulsen zur kinästhetischen Stimulation zu erzeugen ;
- mindestens einen Effektor (20), der geeignet ist, auf eine externe Hautstelle des Patienten aufgebracht zu werden, und der einen elektromechanischen Wandler umfasst;
- Sensormittel, die geeignet sind, einen Schlafzustand des Patienten zu bestimmen; und
- Mittel zur adaptiven Steuerung des Generators, die nur bei Vorliegen eines bestimmten Schlafzustands durch die Sensormittel bedingt aktiviert werden.; und
- Diskriminatormittel, die geeignet sind, das Schlafstadium des Patienten aus mehreren vorbestimmten Stadien der Gruppe zu bestimmen (30-52), die besteht aus: Langsamwellenschlaf I, Langsamwellenschlaf II, Langsamwellenschlaf III, Langsamwellenschlaf IV und REM-Schlaf,
**dadurch gekennzeichnet, dass** der Effektor ein kinästhetischer Effektor (20) ist, wobei der elektromechanische Wandler ein vibrierender Wandler ist, der geeignet ist, die von dem Generator erzeugten Impulse zu empfangen und eine gegebene kinästhetische Stimulationsenergie abzugeben,
und dass die Mittel zur adaptiven Steuerung des Generators umfassen :
- Modulatormittel, die geeignet sind, den Pegel der kinästhetischen Stimulationsenergie der vom Generator erzeugten Bursts in Abhängigkeit vom Schlafstadium des Patienten, das von den Diskriminatormitteln bestimmt wird, zu modifizieren (70-72).

2. Vorrichtung nach Anspruch 1, wobei die Modulatormittel geeignet sind, die Stimulationsenergie für ein tieferes Schlafstadium zu erhöhen in der aufsteigenden Reihenfolge der aufeinanderfolgenden Stadien I, H, III, IV und REM und umgekehrt.

3. Vorrichtung nach Anspruch 1, die außerdem Folgendes umfasst:
- Mittel zur Erkennung (68) des Auftretens einer Apnoe- oder Hypopnoe-Episode; und
- Mittel, die den Konditionierungsgenerator bei Erkennung einer Apnoe- oder Hypopnoe-Episode aktivieren können.

4. Vorrichtung nach Anspruch 3, die außerdem umfasst:
- Mittel (68) zum Erkennen des Endes der Apnoe- oder Hypopnoe-Episode; und
- Mittel, die geeignet sind, den Generator bei Erkennung des Endes der Apnoeoder Hypopnoe-Episode zu deaktivieren.

5. Vorrichtung nach Anspruch 1, bei der die Modulatormittel Mittel umfassen, die geeignet sind, ein vorbestimmtes Inkrement/Dekrement der Stimulationsenergie anzuwenden, das von jedem der vorbestimmten Schlafstadien abhängt.

6. Vorrichtung nach Anspruch 1, bei der die Modulatormittel eine Zuordnungstabelle umfassen, die jedem der vorbestimmten Schlafstadien einen Energie-pegel eines vorbestimmten Stimulationsmusters zuordnet.

7. Vorrichtung nach Anspruch 6, die außerdem Initialisierungsmittel (56) umfasst, die geeignet sind, bei Bestimmung eines Schlafzustands durch Sensormitteln, die Energiepegelwerte der Zuordnungstabelle zu setzen.

8. Vorrichtung nach Anspruch 1, die außerdem Folgendes umfasst:
- Evaluierungsmittel, die geeignet sind, eine autonome Reaktion des Patienten auf die kinästhetische Stimulation in Abhängigkeit von der Veränderung zu bestimmen, (z. B. bei der Erzeugung des Impulsbündels) eines Parameters, der die aktuelle autonome Aktivität des Patienten anzeigt.

9. Vorrichtung nach Anspruch 8, wobei der Parameter, der die aktuelle autonome Aktivität des Patienten anzeigt, die laufende Herzfrequenz (HR) des Patienten ist.

10. Vorrichtung nach Anspruch 8, wobei der Parameter, der die aktuelle autonome Aktivität des Patienten anzeigt, ein Parameter aus der folgenden Gruppe ist: Atemfluss; Sauerstoffsättigung des Blutes; und ein Parameter, der von einem phonokardiographischen oder endokardiographischen Beschleunigungssignal abgeleitet sind.

11. Vorrichtung nach Anspruch 8, bei der die Initialisierungsmittel (56) zur Festlegung des dem ersten Schlafstadium entsprechenden Wertes des Energiepegels Folgendes umfassen:
- Mittel, die geeignet sind, die Stimulationsenergie auf einen vorbestimmten Standard-Minimalwert zu initialisieren (60) ;
- Mittel, die geeignet sind, die aktuelle autonome Reaktion, die von Seinen Evaluationsmitteln bestimmt wird, mit einem ersten Schwellenwert zu vergleichen (62) ;
- Mittel, die geeignet sind, wenn die aktuelle autonome Reaktion unter dem ersten Schwellenwert liegt, die Stimulationsenergie schrittweise und iterativ zu verändern (64), bis der erste Schwellenwert überschritten wird; und
- Mittel, die geeignet sind, für das erste Schlafstadium den Wert der Stimulationsenergie zu speichern (66), der nach dem Überschreiten des Schwelle ermittelt wird.

12. Vorrichtung nach Anspruch 11, ferner umfassend Mittel, die geeignet sind, Stimulationsenergiewerte zu berechnen (66) und zu speichern, die den Schlafstadien entsprechen, die auf das erste Stadium folgen, je nach den gespeicherten Stimulationsenergiewert für das erste Stadium.

13. Vorrichtung nach Anspruch 8, bei der die Diskriminatormittel geeignet sind, das aktuelle Schlafstadium in Abhängigkeit von der durch die Evaluatormittel ermittelten autonomen Reaktion zu bestimmen (30-52).

## Claims

1. A device for treating the sleep apnea syndrome in a patient comprising :
- a generator (18) adapted to generate bursts of controlled pulses for kinaesthetic stimulation ;
- at least one effector (20) adapted to be applied to an external skin site of the patient and comprising an electromechanical transducer;
- sensor means capable of determining a sleep state of the patient; and
- means for adaptive control of the generator, which is conditionally activated by the sensing means only in the presence of a particular sleep condition; and
- discriminant means suitable for determining the patient's sleep stage from several predetermined stages of the group (30-52), which consists of: Slow wave sleep I, Slow wave sleep II, Slow wave sleep III, Slow wave sleep IV and REM sleep,
**characterised in that** the effector is a kinaesthetic effector (20), the electromechanical transducer being a vibrating transducer adapted to receive the pulses generated by the generator and to deliver a given kinaesthetic stimulation energy, and **in that** the means for adaptively controlling the generator comprise :
- modulator means capable of modifying the level of kinesthetic stimulation energy of the bursts produced by the generator as a function of the patient's sleep stage determined by the discriminator means (70-72).

2. The apparatus of claim 1, wherein said modulator means is adapted to increase the stimulation energy for a deeper sleep stage in the ascending order of successive stages I, H, III, IV and REM and vice versa.

3. The apparatus of claim 1, further comprising:
- means for detecting (68) the occurrence of an apnea or hypopnea episode; and
- means that can activate the conditioning generator upon detection of an apnea or hypopnea episode.

4. The apparatus of claim 3, further comprising :
- means (68) for detecting the end of the apnea or hypopnea episode; and
- means capable of deactivating the generator upon detection of the end of the apnea or hypopnea episode.

5. The apparatus of claim 1 wherein said modulator means comprises means adapted to apply a predetermined increment/decrement of stimulation energy dependent upon each of said predetermined sleep stages.

6. The apparatus of claim 1, wherein the modulator means comprises an assignment table that assigns an energy level of a predetermined stimulation pattern to each of the predetermined sleep stages.

7. The apparatus of claim 6, further comprising initialization means (56) adapted, upon determination of a sleep state by sensor means, to set the energy level values of the allocation table.

8. The apparatus of claim 1, further comprising:
- evaluation means suitable for determining an autonomic response of the patient to the kinaesthetic stimulation as a function of the variation, (e.g. in the generation of the impulse beam) of a parameter indicative of the current autonomic activity of the patient.

9. The device of claim 8, wherein the parameter indicative of the patient's current autonomic activity is the patient's running heart rate (HR).

10. The device of claim 8, wherein the parameter indicative of the current autonomic activity of the patient is a parameter selected from the group consisting of: respiratory flow; blood oxygen saturation; and a parameter derived from a phonocardiographic or endocardiographic acceleration signal.

11. The apparatus of claim 8, wherein the initialization means(56) for determining the value of the energy level corresponding to the first sleep stage comprises:
- means adapted to initialize the stimulation energy to a predetermined standard minimum value (60) ;
- means appropriate to compare the current autonomic response, determined by its means of evaluation, with an initial threshold (62) ;
- means adapted, when the current autonomic response is below the first threshold, to vary (64) the stimulation energy gradually and iteratively until the first threshold is exceeded; and
- means suitable for storing, for the first stage of sleep, the value of the stimulation energy (66) determined after the threshold has been crossed.

12. The apparatus of claim 11, further comprising means adapted to calculate (66) and store stimulation energy values corresponding to sleep stages following the first stage, depending on the stored stimulation energy value for the first stage.

13. The apparatus of claim 8, wherein the discriminator means is adapted to - determine the current sleep stage as a function of the autonomic response determined by the evaluator means (30-52).
